# NEUE EUROPÄISCHE PATENTSCHRIFT

(11) **EP 0 299 914 B2**
(45) Veröffentlichungstag und Bekanntmachung des Hinweises auf die Entscheidung über den Einspruch: **23.04.2003**
(45) Hinweis auf die Patenterteilung: 07.04.1993
(21) Anmeldenummer: 88730162.0
(22) Anmeldetag: 15.07.1988
(51) Int. Cl.: C07C 405/00, A61K 31/557

(54) **9-Halogen-(Z)-prostaglandinderivate, Verfahren zu ihrer Herstellung und ihre Verwendung als Arzneimittel**
9-Halogen (Z) prostaglandin derivatives, process for their production and their use as medicines
Dérivés de prostaglandines 9-halogène (Z), leur procédé de préparation et leur application thérapeutique

(30) Priorität: 17.07.1987 DE 3724189; 17.07.1987 DE 3724190
(43) Veröffentlichungstag der Anmeldung: 18.01.1989
(73) Patentinhaber: SCHERING AKTIENGESELLSCHAFT, 13342 Berlin (DE)
(72) Erfinder: Buchmann, Bernd, Dr., D-1000 Berlin 21 (DE); Skuballa, Werner, Dr., D-1000 Berlin 28 (DE); Vorbrüggen, Helmut, Prof. Dr., D-1000 Berlin 27 (DE); Radüchel, Bernd, Dr., D-1000 Berlin 28 (DE); Loge, Olaf, Dr., D-1000 Berlin 27 (DE); Elger, Walter, Dr., D-1000 Berlin 33 (DE); Stürzebecher, Claus-Steffen, Dr., D-1000 Berlin 46 (DE); Thierauch, Karl-Heinz, Dr., D-1000 Berlin 37 (DE)

(56) Entgegenhaltungen:
- EP-A- 0 011 591
- EP-A- 0 030 377
- EP-A- 0 055 208
- EP-A- 0 069 696
- WO-A-86/05488
- WO-A-94/02457
- DE-A- 3 724 189
- DE-A- 3 724 190
- DE-A- 4 229 048
- DE-A- 4 229 053
- GB-A- 1 269 657
- US-A- 3 936 487
- US-A- 3 980 694
- US-A- 4 579 958
- J. of Med. Chem., Bd. 29, Nr. 3, S. 312-315, 1986
- Proceedings of B.P.S., 15.-16.7. 1976, S. 297-298
- Information Material-I=Advances in Prostaglandin, Thromboxane and Leukotriene Research, Bd. 21, Herausg. B. Sameulsson et al, Raven Press, Ltd., New York, US, S. 591-594, 1990
- Artikel aus Prostacyclin, Herausg. J.R. Vane et al, Raven Press, New York, US, S. 17-29, 1979
- Annual Reports in Med. Chem., Bd. 11, S. 80, 81, 86, 1976
- J. S. Skotnicki et al, J. Med. Chem., Bd. 20, Nr. 12, S. 1551-1557, 1977
- Houben-Weyl, Methoden der org. Chemie, Bd. V, 1b, S. 589, 1. Absatz
- Recueil, 86, 1138, 1967
- Zh. O. KH., Bd. 34, Nr. 5, 1389, 1964
- G. Bundy et al, Ann. N.Y. Acad. Sci., 180, 1971
- N.A. Nelson et al, Abstract, International Conference on Prostaglandins, Florence, S. 72, 1975
- E.W. Yankee et al, Advances in Prostaglanding and Thromboxane Research, Bd. 1, S. 195-203, 1976
- G.L. Bundy et al, J. Med. Chem., 26, S. 790-799, 1983
- S.S. Tynan et al, Prostaglandins, Bd. 27, Nr. 5, S. 683-696, Mai 1984
- H. Wieland et al, Annalen der Chemie, Bd. 470, S. 201-223, 1929
- K.-H. Thierauh et al, Prostaglandins, Bd. 35, Nr. 6, S. 855-868, Juni 1988

## Beschreibung

Die vorliegende Erfindung betrifft neue 9-Halogen-(Z)-prostaglandinderivate. Verfahren zu ihrer Herstellung sowie ihre Verwendung als Arzneimittel.

Aus EP-A-0 030 377 sind 9-Chlorprostaglandinderivate und aus EP-A-0 069 696 9-Fluorprostaglandinderivate bekannt, die in 5-Stellung der oberen Seitenkette eine Doppelbindung tragen. Sie wirken lyteolytisch, hemmen die Magensäuresekretions und zeigen zytoprotektive und ulcusheilende Effekte. Außerdem wirken sie blutdrucksenkend und hemmen die Plättchenaggregation.

Mit den 9-Halogenprostaglandinen aus WO 86/05488 sind Verbindungen bekannt, die ein analoges Wirkspektrum aufweisen.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

Es wurde nun gefunden, daß die neuen 9-Halogen-(Z)-prostaglandinderivate eine hervorragende Wirkungsspezifität, eine bessere Wirksamkeit und eine längere Wirkungsdauer als natürliche Prostaglandine und deren Derivate besitzen und besonders für die orale Applikation geeignet sind.

Die Erfindung betrifft 9-Halogen-(Z)-prostaglandinderivate der Formel I 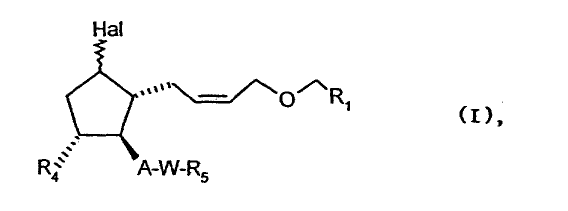 worin
- Hal: ein α- oder β-ständiges Chlor- oder Fluoratom ist,
- R₁: den Rest CH₂OH oder 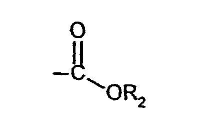
bedeutet, mit R₂ in der Bedeutung eines Wasserstoffatoms, eines Alkyl-, Cycloalkyl-, Aryl- oder heterocyclischen Restes oder R₁ den Rest 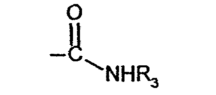 bedeutet, mit R₃ in der Bedeutung eines Säurerestes oder des Restes R₂ und
- A: eine -CH₂-CH₂-, eine trans-CH = CH- oder eine -C≡C-Gruppe ist.
- W: eine freie oder funktionell abgewandelte Hydroxymethylengruppe oder eine freie oder funktionell abgewandelte 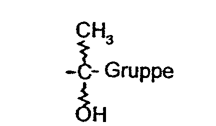
bedeutet, wobei die jeweiligen OH-Gruppen α- oder β-ständig sein können.
- R₄: eine freie oder funktionell abgewandelte Hydroxygruppe ist,
- R₅: eine Cycloalkylgruppe ist, die 3-10 Kohlenstoffatome im Ring enthält, und falls R₂ die Bedeutung eines Wasserstoffatoms hat, deren Salze mit physiologisch verträglichen Basen bedeutet und deren Cyclodextrinchlathrate.

Als Alkylgruppen R₂ und R₁₀ sind gerade oder verzweigte Alkylgruppen mit 1-10 C-Atomen zu betrachten, wie beispielsweise Methyl, Ethyl, Propyl, Butyl. Isobutyl, tert.-Butyl, Pentyl, Neopentyl, Hexyl, Heptyl, Decyl. Die Alkylgruppen R₂ und R₁₀ können gegebenenfalls ein- bis mehrfach substituiert sein durch Halogenatome, Alkoxygruppen, gegebenenfalls substituierte Aryl- bzw. Aroylgruppen, Dialkylamino und Trialkylammonium, wobei die einfache Substitution bevorzugt sein soll. Als Substituenten seien beispielsweise genannt Fluor, Chlor oder Brom, Phenyl, Dimethylamino, Diethylamino, Methoxy, Ethoxy. Als bevorzugte Alkylgruppen R₂ und R₁₀ sind solche mit 1-4 C-Atomen, wie z.B. Methyl, Ethyl, Propyl, Dimethylaminopropyl, Isobutyl, Butyl zu nennen.

Als Arylgruppen R₂ und R₁₀ kommen sowohl substituierte als auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Cycloalkylgruppe R₂ und R₁₀ kann im Ring 3-10, vorzugsweise 5 und 6 Kohlenstofatome enthalten. Die Ringe können durch Alkylgruppen mit 1-4 Kohlenstoffatomen substituiert sein. Beispielsweise seien genannt Cyclopentyl, Cyclohexyl und Methylcyclohexyl.

Als heterocyclische Gruppen R₂ und R₁₀ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest R₃ kommen physiologisch verträgliche Säurereste in Frage. Als Säuren sind organische Carbonsäuren und Sulfonsäuren mit 1-15 Kohlenstoffatomen geeignet, die der aliphatischen, cycloaliphatischen, aromatischen, aromatischaliphatischen und heterocyclischen Reihe angehören. Diese Säuren können gesättigt, ungesättigt und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure. Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Ethoxyessigsäure, Mono-, Di- und Trichloressigsäure, Aminoessigsäure, Diethylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy-oder Carboxy-Gruppen substituierte Benzoesäuren, Nikotinsäure, Isonikotinsäure, Furan-2-carbonsäure, Cyclopentylpropionsäure. Als bevorzugte Acylreste werden solche mit bis zu 10 Kohlenstoffatomen betrachtet. Als Sulfonsäuren kommen beispielsweise Alkansulfonsäuren mit 1-10 C-Atomen wie z.B. Methansulfonsäure, Ethansulfonsäure, Isopropansulfonsäure und Butansulfonsäure sowie β-Chlorethansulfonsäure, Cyclopentansulfonsäure, Cyclohexansulfonsäure, Benzolsulfonsäure, p-Toluolsulfonsäure, p-Chlorbenzolsulfonsäure, N.N-Dimethylaminosulfonsäure,N.N-Diethylaminosulfonsäure, N.N-Bis(β-chlorethyl)-aminosulfonsäure, N.N-Diisobutylaminosulfonsäure, N.N-Dibutylaminosulfonsäure, Pyrrolidino-, Piperidino-, Piperazino-, N-Methylpiperazino- und Morpholinosulfonsäure in Frage. Besonders bevorzugt sind Acylreste bzw. Alkansulfonsäurereste mit 1-4 C-Atomen.

Die Hydroxygruppen in W und R₄ können funktionell abgewandelt sein, beispielsweise durch Veretherung oder Veresterung, wobei auch die abgewandelte Hydroxygruppe in W α- oder β-ständig sein kann, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, α-Ethoxyethyl-, Trimethylsilyl-, Dimethyl-tert.-butyl-silyl-, Dimethyl-thexyl-silyl-, Diphenyl-tert.-butyl-silyl- und Tribenzyl-silylrest. Als Acylreste kommen die gleichen wie für R₃ unter organischen Carbonsäuren genannt in Frage, namentlich genannt seien beispielsweise Acetyl, Propionyl, Butyryl und Benzoyl.

Die Cycloalkylgruppe R₅ kann im Ring 3-10, vorzugsweise 3-6 Kohlenstoffatome enthalten. Die Ringe können durch Alkylgruppen mit 1-4 Kohlenstoffatomen substituiert sein. Beispielsweise genannt seien Cyclopropyl, Cyclobutyl, Cyclopentyl, Cyclohexyl und Methyl-cyclohexyl.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natriumund Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Erfindung betrifft außerdem ein Verfahren zur Herstellung der erfindungsgemäßen 9-Halogen-(Z)-prostaglandinderivate der Formel I, dadurch gekennzeichnet, daß man in an sich bekannter Weise eine Verbindung der Formel II 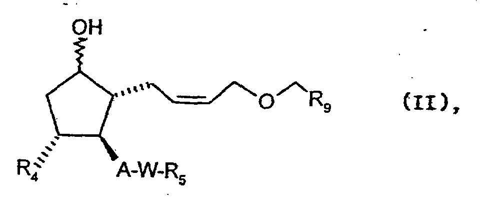 worin die 9-OH-Gruppe α-oder β-ständig sein kann und
- R₉: den Rest 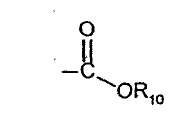 mit R₁₀ in der Bedeutung von Alkyl, Cycloalkyl, Aryl oder heterocyclischer Rest oder den Rest 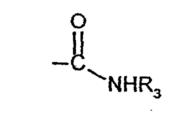 mit R₃ in der Bedeutung eines Säurerestes, eines Alkyl-, Cycloalkyl-, Aryl- oder eines heterocyclischen Restes darstellt und A und R₅ die bereits oben angegebenen Bedeutungen haben, nach vorherigem Schutz freier OH-Gruppen in R₄ und W
a) über einen intermediären Sulfonsäureester mit einem Halogenid der allgemeinen Formel III,

   R₈―X (III),

   worin R₈ die Bedeutung Lithium, Natrium, Kalium oder Tetraalkyl- bzw. Trialkylbenzyl-ammonium mit Alkyl als gesättigten C₁-C₆-Rest und X die Bedeutung Fluor oder Chlor hat oder
b) mit dem Reagenz Diethylaminoschwefeltrifluorid (DAST) zu Verbindungen der Formel I, worin Hal ein α- oder β-ständiges Fluoratom ist oder mit Tetrachlorkohlenstoff bzw. Hexachlorethan/Triphenylphosphin zu Verbindungen der Formel I, worin Hal ein α- oder β-ständiges Chloratom ist, umsetzt und anschließend in beliebiger Reihenfolge geschützte Hydroxygruppen freisetzt und/oder freie Hydroxygruppen verestert oder verethert und/oder Doppelbindungen hydriert und/oder eine veresterte Carboxylgruppe 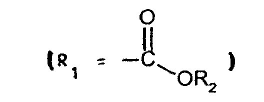 verseift und/oder eine freie Carboxylgruppe (R₂ = H) in ein Amid 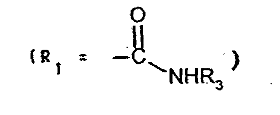 oder Salz überführt und/oder eine freie oder veresterte Carboxylgruppe 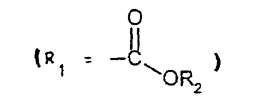 reduziert.

Die Umsetzung der Verbindungen der Formel II zu den Verbindungen der Formel I erfolgt zunächst durch Überführung mit einem Sulfonsäurechlorid oder Sulfonsäureanhydrid in einen Sulfonsäureester in der für den Fachmann bekannten Weise und anschließender Umsetzung mit einem Halogenid der Formel III in einem inerten Lösungsmittel wie beispielsweise Benzol, Toluol, Diethylether, Tetrahydrofuran, Methylenchlorid, Acetonitril, Dimethylformamid bei Temperaturen zwischen 0°C und 100°C, vorzugsweise 20°C bis 70°C.

Die Unmsetzung der Verbindungen der Formel II zu den Verbindungen der Formel I mit Tetrachlorkohlenstoff und Triphenylphosphin oder Hexachlorethan/Triphenylphosphinerfolgt in einem inerten Lösungsmittel wie beispielsweise Dimethylformamid, Dimethylacetamid, Acetonitril, Methylenchlorid bei Temperaturen zwischen 0°C und 80°C, vorzugsweise 20°C bis 45°C in Gegenwart einer Base wie beispielsweise Pyridin, Triethylamin usw.

Die Umsetzung der Verbindungen der Formel II zu den Verbindungen der Formel I mit Hal in der Bedeutung eines Fluoratoms erfolgt mit Diethylaminoschwefeltrifluorid in einem Lösungsmittel wie beispielsweise Dichlormethan bei Temperaturren zwischen -120°C und 0°C, vorzugsweise bei -70 °C, gegebenenfalls in Gegenwart einer tertiären Base wie beispielsweise Pyridin.

Setzt man einen Alkohol der Formel II mit einer β-ständigen 9-Hydroxygruppe ein, so erhält man Verbindungen der Formel I mit 9-α-ständigem Halogenatom, setzt man einen Alkohol mit einer α-ständigen Hydroxygruppe ein, so erhält man Verbindungen mit 9-β-ständigem Halogenatom.

Die Reduktion zu den Verbindungen der Formel I mit R₁ in der Bedeutung einer -CH₂OH-Gruppe wird mit einem für die Reduktion von Estern oder Carbonsäuren geeigneten Reduktionsmittel wie beispielsweise Lithiumaluminiumhydrid, Diisobutylaluminiumhydrid usw. durchgeführt. Als Lösungsmittel kommen Diethylether, Tetrahydrofuran, Dimethoxyethan, Toluol usw. in Frage. Die Reduktion wird bei Temperaturen von -30°C bis zur Siedetemperatur des verwendeten Lösungsmittels, vorzugsweise 0°C bis 30°C vorgenommen.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wässrigen Lösung einer organischen Säure, wie z.B. Oxalsäure, Essigsäure, Propionsäure u.a., oder in einer wässrigen Lösung einer anorganischen Säure, wie z.B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z.B. Alkohole, wie Methanol und Ethanol, und Ether, wie Dimethoxyethan, Dioxan und Tetrahydrofuran, Tetrahydrofuran wird bevorzugt angewendet. Die Abspaltung wird vorzugsweise bei Temperaturen zwischen 20 °C und 80°C durchgeführt.

Die Verseifung der Acylgruppen erfolgt beispielsweise mit Alkali- oder Erdalkali-carbonaten oder -hydroxyden in einem Alkohol oder in der wässrigen Lösung eines Alkohols. Als Alkohole kommen aliphatische Alkohole in Betracht, wie z.B. Methanol, Ethanol, Butanol usw. vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und Bariumcarbonat. Die Umsetzung erfolgt bei -10°C bis +70°C, vorzugsweise bei +25°C.

Die Einführung der Estergruppe 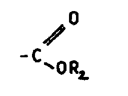 für R₁, bei welcher R₂ eine Alkylgruppe mit 1-10 C-Atomen darstellt, erfolgt nach den dem Fachmann bekannten Methoden. Die Carboxy-Verbindungen werden beispielsweise mit Diazokohlenwasserstoffen in an sich bekannter Weise umgesetzt. Die Veresterung mit Diazokohlenwasserstoffen erfolgt z. B dadurch, daß man eine Lösung des Diazokohlenwasserstoffes in einem inerten Lösungsmittel, vorzugsweise in Diethylether, mit der Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8. Seiten 389-394 (1954)].

Die Einführung der Estergruppe 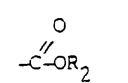 für R₁, bei welcher R₂ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel ungesetzt. Als Lösungsmittel kommen Methylenchlorid, Ethylenchlorid, Chloroform in Frage. Die Reaktion wird bei Temperaturen zwischen -30 °C und +50 °C, vorzugsweise bei 10 °C, durchgeführt.

Die Prostaglandinderivate der Formel I mit R₂ in der Bedeutung eines Wasserstoffatoms können mit geeigneten Mengen der entsprechenden anorganischen Base unter Neutralisierung in ein Salz überführt werden. Beispielsweise erhält man beim Lösen der entsprechenden PG-Säuren in Wasser, das die stöchiometrische Menge der Base enthält, nach Abdampfen des Wassers oder nach Zugabe eines mit Wasser mischbaren Lösungsmittels. z.B. Alkohol oder Aceton, das feste anorganische Salz.

Zur Herstellung eines Aminsalzes, die in üblicher Weise erfolgt, wird die PG-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert. Die Einführung der Amidgruppe 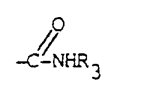 für R₁ erfolgt nach den dem Fachmann bekannten Methoden. Die Carbonsäuren der Formel I (R₂ = H), werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amids oder mit Ammoniak (R₃ = H) oder des entsprechenden Amins erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30 °C und +60 °C, vorzugsweise bei 0 °C bis 30 °C.

Eine weitere Möglichkeit für die Einführung der Amidgruppe 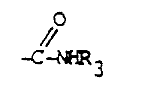 für R₁ mit R₃ in der Bedeutung eines Säurerestes besteht in der Umsetzung einer 1-Carbonsäure der Formel I (R₂ = H), in der freie Hydroxygruppen gegebenenfalls intermediär geschützt sind, mit Verbindungen der Formel IV

O = C = N - R₃ (IV)

worin R₃ die obenangegebene Bedeutung hat.

Die Umsetzung der Verbindung der Formel I (R₂ = H) mit einem Isocyanat der Formel IV folgt gegebenenfalls unter Zusatz eines tertiären Amins, wie z.B. Triethylamin oder Pyridin. Die Umsetzuug kann ohne Lösungsmittel oder in einem inerten Lösungsmittel, vorzugsweise Acetonitril, Tetrahydrofuran, Aceton, Dimethylacetamid, Methylenchlorid, Diethylether, Toluol, bei Temperaturen zwischen -80 °C bis 100 °C, vorzugsweise bei 0 bis 30 °C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Ether- oder Acylreste intermediär geschützt sind.

Die als Ausgangsmaterial dienenden Verbindungen der Formel II mit einer 9α-Hydroxygruppe können beispielsweise hergestellt werden, indem man in an sich bekannter Weise ein Lacton der Formel V 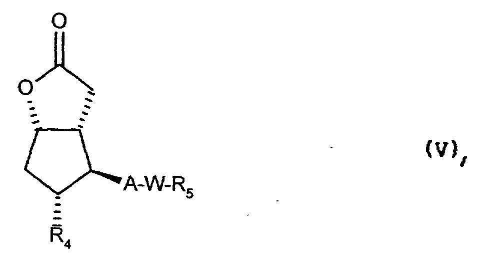 worin A und R₅ die bereits oben angegebenen Bedeutungen haben und die in R₄ und W vorhandenen OH-Gruppen mit einer basisch-resistenten Schutzgruppe, wie z.B. durch Veretherung mit Dihydropyran, versehen sind, durch Behandlung mit Basen, wie beispielsweise Natriumhydroxid, und anschließendem vorsichtigen Ansäuern in die Hydroxysäuren der Formel VI überführt.

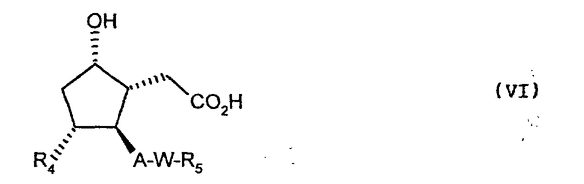

Nach Veresterung der Säure mit Diazomethan und Veretherung der freien OH-Gruppe mit Dimethyltert.-butylsilylchlorid wird der Ester in der für den Fachmann bekannten Weise entweder direkt zu den Aldehyden der Formel VII, 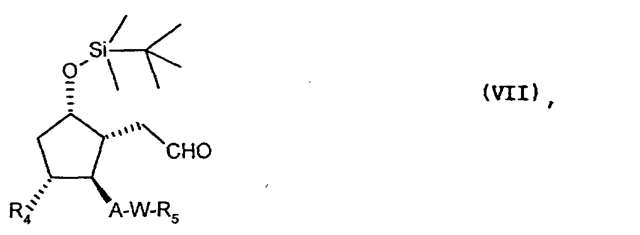 reduziert oder zunächst zu den entsprechenden Alkoholen reduziert und anschließend zu den Aldehyden der Formel VII oxidiert.

Durch Reaktion dieser Aldehyde mit Tetrabrommethan/Triphenylphosphin in Gegenwart von Zink und Behandlung der hieraus resultierenden Rohprodukte mit Butyllithium gelangt man zu den Alkinen der Formel VIII.

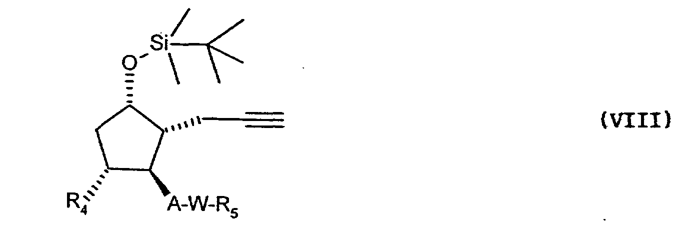

Nach Metallierung der Alkine der Formel VIII beispielsweise mit Butyllithium und Umsetzung mit Formaldehyd werden die so erhaltenen Propargylalkohole unter basischen Bedingungen mit Bromessigsäure-tert.-butylester verethert und liefern die Ester der Formel IX.

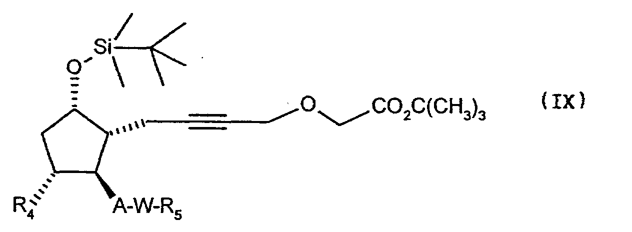

Durch Lindlar-Hydrierung der Alkinester der Formel IX und anschliessende selektive Abspaltung der Silylschutzgruppe in der 9-Position erhält man die als Ausgangsmaterial dienenden Verbindungen der Formel II mit einer 9α-Hydroxygruppe.

Die Verbindungen der Formel II mit einer 9β-Hydroxygruppe erhält man aus den 9α-Hydroxyverbindungen durch eine Inversionsreaktion, wie sie z.B. in Synthesis, 292-294 (1980) beschrieben wurde.

Im Vergleich zu PGE-Derivaten zeichnen sich die neuen Prostaglandinanaloga durch größere Stabilität aus.

Die neuen Prostaglandinanaloga der Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die erfindungsgemäßen Wirkstoffe zeigen einen zytoprotektiven und ulkusheilenden Effekt, hemmen die Magensäuresekretion und wirken damit den unerwünschten Folgen nichtsteroidaler Entzündungshemmstoffe entgegen. Sie wirken außerdem an der Leber, Niere und auch an der Bauchspeicheldrüse zytoprotektiv.

Die neuen Prostaglandin-Analoga wirken stark luteolytisch, d.h. zur Auslösung einer Luteolyse benötigt man wesentlich geringere Dosierungen als bei den entsprechenden natürlichen Prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, daß die erfindungsgemäßen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemäßen Prostaglandin-Derivate zur Cervixdilatation als Vorbereitung für diagnostische oder therapeutische Eingriffe.

Die gute Gewebsspezifität der erfindungsgemäßen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemäßen Substanzen wirken auch bronchospasmolytisch. Außerdem bewirken sie eine Abschwellung der Nasenschleimhaut.

Einige der Verbindungen wirken blutdrucksenkend, regulierend bei Herzrhythmusstörungen und hemmend auf die Plättchenaggregation mit den sich daraus ergebenden Einsatzmöglichkeiten, wie z.B. bei koronarer Herzkrankheit und Herzinfarkt. Die neuen Prostagtandine können auch in Kombination, z.B. mit β-Blockern, Diuretika, Phosphodiesterasehemmern, Calciumantagonisten, Thromboxanantagonisten, Thromboxansynthetase- und Cyclooxygenasehemmern, gerinnungshemmenden Substanzen, wie auch Fibrinolytika, Leukotrienantogonsiten, Leukotriensynthetasehemmern und Antigestagenen, verwendet werden.

Im Vergleich zu ³H-PGD₂ zeigte (5Z,13E)-(9R,11R,15S)-9-Chlor-3-oxa-15-cyclohexyl-11,15-dihydroxy-16,17,18,19,20 -pentanor-5,13-prostadiensäure (A) im Rezeptortest einen Kompetitionsfaktor von 0,5. Außerdem senkte die Verbindung A den Blutdruck nach i.V.-Gabe im Vergleich zu (5Z,13E)-(9R,11R,15S)-9-Chlor-15-cyclohexyl-11,15-dihydroxy-16,17,18,19,20-pentanor-5,13-prostadlensäure aus WO 86/05488 mehr als doppelt so gut.

Die neuen Prostaglandinanaloga besitzen eine hohe Affinität für Rezeptoren in Membranpräparationen aus Gehirn und können in Folge ihrer Eigenschaften zur Beeinflussung psychischer Prozesse, wie z.B. Schlaf dienen.

Die Dosis der Verbindung ist 1-1500 µg/kg/Tag, wenn sie am menschlichen Patienten verabreicht werden.

Für die medizinische Anwendung können die Wirkstoffe in eine für die Inhalation, für orale, parenterale oder lokale (z.B. vaginale) Applikation geeignete Form überführt werden. Zur Inhalation werden zweckmäßigerweise Ärosollösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt.

Für die vaginale Applikation sind z.B. Zäpfchen geeignet und üblich.

Die Erfindung betrifft damit auch Arzneimittel auf Basis der Verbindungen der Formel I und üblichen Hilfs- und Trägerstoffe, einschließlich Cyclodextrinclathraten.

Die erfindungsgemäßen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt, zur Behandlung der Hypertonie oder zur Behandlung von gastrointestinalen Störungen, wie z.B. zur Abheilung von Magen- und Zwölffingerdarmgeschwüren, dienen. Für diesen Zweck aber auch für die übrigen Anwendungen können die Präparate 0,01 - 100 mg der aktiven Verbindung enthalten.

Die folgenden Beispiele sollen die Erfindung näher erläutern, ohne daß damit eine Begrenzung vorgenommen wird.

### Beispiel 1

### (5Z,13E)-(9R,11R,15S)-9-Chlor-15-cyclohexyl-11,15-dihydroxy-3 -oxa-16,17,18,19,20-pentanor-5,13-prostadiensäure-tert.-butytester

Zu einer Lösung von 2,54 g (5Z,13E)-(9S,11R,15S)-15-Cyclohexyl-9-hydroxy-3-oxa-11,15-bis-(tetrahydropyran-2-yloxy)-16,17,18,19,20-pentanor-5,13-prostadiensäure-ter.-butylester in 25 ml Pyridin gibt man bei 0°C 773 mg Methansulfonsäurechlorid. Man rührt 4 Stunden bei 20° C und gibt dann die Lösung zu einer Suspension von 25,37 g Tetrabutylammoniumchlorid in 25 ml Toluol. Nach 15 stündigem Rühren bei 0°C wird noch 7 Stunden bei 40° C gerührt. Anschließend gibt man auf 100 ml Eiswasser und extrahiert dreimal mit je 50 ml Ether. Nachdem die organische Phase je zweimal mit 20 ml Sole gewaschen, über MgSO₄ getrocknet und im Vakuum eingedampft wird, erhält man einen Rückstand, den man an Kieselgel mit Hexan/0-40 % Ether chromatographiert. Man erhält 2,32 g öligen (5Z,13E)-(9R,11R,15S)-9-Chlor-15-cyclohexyl-3-oxa-11,15-bis-(tetrahydropyran-2-yloxy)-16,17,18,19,20-pentanor-5,13-prostadiensäure-tert.butylester. Zur Abspaltung der Schutzgruppen rührt man den erhaltenen Ester mit 90 ml einer Mischung aus Essigsäure/Wasser/Tetrahydrofuran (65/35/10) 24 Stunden bei 20°C. Nach Zugabe von Toluol und Eindampfen der Lösung im Vakuum chromatographiert man den Rückstand an Kieselgel. Mit Methylenchlorid/0,5 % Aceton als Elutionsmittel erhält man 915 mg der Titelverbindung als farbloses Öl. IR(CHCl₃): 3605, 3410, 2928, 1742, 1020, 974/cm.

Der als Ausgangsmaterial verwendete 9α-Alkohol wird wie folgt erhalten:

### 1a) (13E)-(9S, 11R, 15S)-15-Cyclohexyl-9 - (tert.-butyldimethylsilyloxy)-11,15-bis- (tetrahydropyran-2-yloxy ) -2, 3, 4, 5, 6, 16, 17, 18, 19, 20-decanor-13-prostensäuremethylester

Zu einer Lösung von 6,45 g (3aR, 4R, 5R, 6aS)-4-[(E)-(3S)-3-Cyclohexyl-3-(tetrahydropyran-2-yloxy)-1-propenyl]-5-(tetrahydropyran-2-yloxy)-perhydrocyclopenta [b] furan-2-on in 56 ml Methanol gibt man 56 ml einer 1 N wäßrigen Natronlauge und rührt 24 Stunden bei 20° C. Anschließend wird der Methanolanteil durch Einengen im Vakuum entefernt und die so erhaltene wäßrige Lösung mit kalter 10 %iger Schwefelsäure auf pH 4,5 eingestellt. Dann extrahiert man zunächst mit 400 ml Methylenchlorid/Ethylacetat (1/1) und anschließend noch zweimal mit je 100 ml Ethylacetat. Die organischen Extrakte werden mit Sole neutral gewaschen, über MgSO₄ getrocknet und im Vakuum eingedampft. Der Rückstand wird in 63 ml Methylenchlorid gelöst, 15 Minuten mit überschüssigem etherischen Diazomethan behandelt und die Lösung zur Trockne eingedampft. Der ölige Rückstand wird in 73 ml Dimethylformamid gelöst und nach Zugabe von 3,51 g Imidazol und 3,88 g tert.-Butyldimethylsilylchlorid 4 Stunden bei 20° C gerührt. Anschließend verdünnt man die Reaktionsmischung mit 600 ml Hexan/Ether (1/1), wäscht mit 100 ml Wasser und dann mit Sole neutral. Man trocknet über MgSO₄, dampft im Vakuum ein und chromatographiert an Kieselgel mit Hexan / 0-30 % Ethylacetat. Man erhält 5,89 g der Titelverbindung als Öl.
IR (CHCl₃): 2930, 1728, 1018, 975/cm.

### 1b) (13E)-(9S, 11R, 15S) -15 -Cyclohexyl -9-(tert. -butyl-dimethylsilyloxy )-11,15-bis- (tetrahydropyran -2 -yloxy ) -2, 3, 4, 5, 6, 16, 17, 18, 19, 20-decanor-13-prostenal

Zu einer Lösung von 5,76 g (13E)-(9S, 11R, 15S)-15-Cyclohexyl-9-(tert.-butyldimethylsilyloxy)-11,15-bis-(tetrahydropyran-2-yloxy)-2, 3, 4, 5, 6, 16, 17, 18, 19, 20-decanor-13-prostensäuremethylester in 240 ml Toluol tropft man bei -70° C 24,2 ml einer 1,2 molaren DIBAH-Lösung in Toluol und rührt noch 2 Stunden bei dieser Temperatur. Anschließend gibt man 3 ml Isopropanol hinzu, rührt für 10 Minuten, um dann 12 ml Wasser zuzutropfen. Nach Entfernen des Kältebades wird nach 3stündigem Rühren bei 20° C vom entstandenen Niederschlag abfiltriert und mit Ethylacetat nachgewaschen. Das Filtrat wird zur Trockne eingedampft und der Rückstand an Kieselgel mit Hexan / 0-40 % Ethylacetat chromatographiert. Man erhält 4,15 g der Titelverbindung als Öl.
IR (CHCl₃): 2930, 2725, 1718, 1020, 972 / cm.

### 1c) (13E)-(9S, 11R, 15S)-15 -Cyclohexyl-9-(tert.-butyldimethylsilyloxy)-11,15-bis - (tetrahydropyran -2 -yloxy)-1, 2, 3, 4, 16, 17, 18, 19, 20-monanor-13-prosten-5-in

Zu einer Suspension von 9,09 g Tetrabrommethan und 1,79 g Zinkpulver in 180 ml Methylenchlorid gibt man bei 20° C 7,20 g Triphenylphosphin und rührt 24 Stunden bei dieser Temperatur. Dann tropft man 3,59 g (13E)-(9S, 11R, 15S)-15-Cyclohexyl-9-(tert.-butyldimethylsilyloxy)-11,15-bis-(tetrahydropyran-2-yloxy)-2, 3, 4, 5, 6, 16, 17, 18, 19, 20-decanor-13-prostenal in 37 ml Methylenchlorid zu und rührt 1,5 Stunden bei 20° C. Anschließend gibt man die Mischung unter Rühren in 2 I Pentan, filtriert und dampft das Filtrat im Vakuum ein. Den öligen Rückstand löst man in 143 ml Tetrahydrofuran, tropft bei -70° C 10,9 ml einer 1,6 molaren-Butyllithium-Lösung in Hexan zu und rührt 1 h bei dieser Temperatur. Dann läßt man auf 20° C erwärmen und rührt nochmals 1 Stunde. Anschließend wird im Vakuum eingeengt, mit 300 ml Ether verdünnt und mit Sole neutral gewaschen. Nach dem Trocknen über Na₂SO₄ wird im Vakuum eingedampft. Der so erhaltene Rückstand wird mit Hexan / 0-20 % Ethylacetat als Elutionsmittel an Kieselgel chromatographiert. Man erhält 4,34 g der Titelverbindung als Öl. IR (CHCl₃): 3210, 2930, 970 / cm.

### 1d) (13E)-(9S, 11R, 15S)-15-Cyclohexyl-9-(tert.-butyldimethylsilyloxy)-11,15-bis- (tetrahydropyran-2-yloxy )-2, 3, 4, 16, 17, 18, 19, 20-octanor-13-prosten-5-inol

Zu einer Lösung von 4,33 g (13E)-(9S, 11R, 15S)-15-Cyclohexyl-9-(tert.-butyldimethylsilyloxy)-11,15-bis-(tetrahydropyran-2-yloxy)-1, 2, 3, 4, 16, 17, 18, 19, 20-nonanor-13-prosten-5-in in 51 ml Tetrahydrofuran tropft man bei -20° C 9,6 ml einer 1,6 molaren Butyllithium-Lösung in Hexan zu und rührt 1 Stunde bei -20° C. Dann gibt man 468 mg getrockneten Paraformaldehyd zu und rührt 90 Minuten bei 0° C. Anschließend verdünnt man mit 50 ml Wasser und extrahiert dreimal mit je 50 ml Ether. Die organischen Extrakte werden zweimal mit je 50 ml Sole gewaschen, über Na₂SO₄ getrocknet und im Vakuum eingedampft. Der Rückstand wird an Kieselgel mit Hexan / 0-30 % Ethylacetat chromatgraphiert. Man erhält 3,11 g der Titelverbindung als Öl. IR (CHCl₃): 3610, 3425, 2930, 1020, 977 / cm.

### 1e) (13E)-(9S, 11R, 15S)-15-Cyclohexyl -3 -oxa -9 -(tert.-butyldimethylsilyloxy)-11,15-bis - (tetrahydropyran -2-yloxy)-16, 17, 18, 19, 20 -pentanor-13 -prosten -5-insäure-tert.-butylester

Zu einer Lösung von 1,72 g (13E)-(9S, 11R, 15S)-15-Cyclohexyl-9-(tert.-butyldimethylsilyloxy)-11,15-bis-(tetrahydroypran-2-yloxy)-2, 3, 4, 16, 17, 18, 19, 20-octanor-13-prosten -5-inol in 22 ml Toluol gibt man 2,84 g Bromessigsäure-tert.-butylester, 8,8 ml 25%ige Natronlauge und 42 mg Tetrabutylammoniumhydrogensulfat. Nach 16stündigem Rühren bei 20° C verdünnt man mit 100 ml Ether und säuert mit Zitronensäure auf pH 6 an. Man extrahiert dreimal mit je 50 ml Ether, wäscht die vereinigten organischen Phasen mit Sole und trocknet über MgSO₄. Nach dem Eindampfen im Vakuum wird der Rückstand an Kieselgel mit Hexan /0-2- % Ethylacetat chromatographiert. Man erhält 1,65 g der Titelverbindung als Öl.
IR (CHCl₃): 2937, 1745, 1020, 977 / cm.

### 1f) (5Z, 13E)-(9S, 11R, 15S)-15 -Cyclohexyl -3 -oxa -9 - (tert. -butyldimethylsilyloxy)-11,15 -bis - (tetrahydropyran-2-yloxy)-16, 17, 18, 19, 20 -pentanor -5,13 -prostadien -säure -tert. -butylester

Eine Mischung aus 1,73 g (13E)-(9S, 11 R, 15S)-15-Cyclohexyl-3-oxa-9-(tert.-butyldimethylsilyloxy)-11,15-bis-(tetrahydropyran-2-yloxy)-16, 17, 18, 19, 20-pentanor-13-prosten -5-insäure-tert.-butylester und 519 mg Lindlar-Katalysator in 480 ml Toluol wird in einer Wasserstoffatmosphäre gerührt. Nach Aufnahme von einem Äquivalent Wasserstoff wird abfiltriert. Nach dem Eindampfen des Filtrats im Vakuum chromatographiert man den Rückstand an Kieselgel. Mit Hexan / 0-20 % Ethylacetat als Elutionsmittel erhält man 1,74 g der Titelverbindung als Öl.
IR (CHCl₃): 2930, 1745, 1020, 976 / cm.

### 1g) (5Z, 13E)-(9S, 11R, 15S)-15-Cyclohexyl -9 -hydroxy -3 -oxa-11,15-bis - (tetrahydropyran -2 -yloxy )-16, 17, 18, 19,20-pentanor-5,13-prostadiensäure-tert.-butylester

3,24 g (5Z, 13E)-(9S, 11R, 15S)-15-Cyclohexyl-3-oxa-9-(tert.-butyldimethylsilyloxy)-11,15-bis-(tetrahydropyran-2-yloxy)-16, 17, 18, 19, 20-pentanor-5,13-prostadiensäure-tert.-butylester werden in 18,3 ml einer 1 molaren Tetrabutylammoniumfluorid-Lösung in Tetrahydrofuran gegeben und 2 Stunden bei 20° C gerührt. Man versetzt mit 150 ml Wasser und extrahiert zweimal mit je 100 ml Methylenchlorid. Die organischen Extrakte werden mit Sole gewaschen, über MgSO₄ getrocknet und im Vakuum eingedampft. Der erhaltene Rückstand wird an Kieselgel mit Hexan / 0-50 % Ethylacetat chromatographiert. Man erhält 2,54 g der Titelverbindung als Öl.
IR (CHCl₃). 3600, 3480, 2930, 1743, 1020, 974 / cm.

### Beispiel 2

### (5Z, 13E)-(9R, 11R, 15S)-9-Chlor-15-cyclohexyl-11,15-dihydroxy-3-oxa-16, 17, 18, 19, 20-pentanor-5,13-prostadiensäure

Zu einer Lösung von 857 mg (5Z, 13E)-(9R, 11R, 15S)-9-Chlor-15-cyclohexyl-11,15-dihydroxy-3-oxa-16, 17, 18, 19, 20-pentanor-5,13-prostadiensäure-tert.-butylester in 7,7 ml Methanol gibt man 7,7 ml einer 0,5 normalen Natronlauge und rührt 18 Stunden bei 20° C. Man verdünnt mit 50 ml Wasser und säuert mit Zitronensäure auf pH 5 an. Man extrahiert dreimal mit je 50 ml Methylenchlorid, trocknet über MgSO₄ und dampft im Vakuum ein. Der Rückstand wird an Kieselgel mit Methylenchlorid / 0-15 % Aceton chromatographiert. Man erhält 75 mg der Titelverbindung als Öl.
IR (CHCl₃): 3605, 3410, 2930, 1738, 1020, 973 / cm.

### Beispiel 3

### (5Z, 13E)-(9S, 11R, 15S)-9-Chlor-15-cyclohexyl-11,15-dihydroxy-3-oxa-16, 17, 18, 19, 20-pentanor-5,13-prostadiensäure

In Analogie zu Beispiel 1 und 2 erhält man aus (5Z, 13E)-(9R, 11R, 15S)-15-Cyclohexyl-9-hydroxy-3-oxa-11,15-bis-(tetrahydropyran-2-yloxy)-16, 17, 18, 19, 20-pentanor-5,13-prostadiensäure-tert.-butylester die Titelverbindung als Öl.
IR (CHCl₃): 3605, 3420, 2930, 1740, 1022, 975 / cm.

Der als Ausgangsmaterial verwendete 9β-Alkohol wird wie folgt erhalten:

### 3a) (5Z, 13E)-(9R, 11R, 15S)-15-Cyclohexyl-9-hydroxy-3-oxa-11,15-bis-(tetrahydropyran-2-yloxy)-16, 17, 18, 19, 20 -pentanor-5,13-prostadiensäure-tert.-butylester

Zu einer Lösung von 2,68 g (5Z, 13E)-(9S, 11R, 15S)-15-Cyclohexyl-9-hydroxy-3-oxa-11,15-bis-(tetrahydropyran-2-yloxy)-16, 17, 18, 19, 20-pentanor-5,13-prostadiensäure-tert.-butylester in 37 ml Pyridin gibt man bei 0° C 1,71 g p-Toluolsulfonsäurechlorid. Nach 1 Stunde entfernt man das Eisbad und läßt 48 Stunden bei 20° C stehen. Man kühlt dann wieder auf 0° C, versetzt mit 0,2 ml Wasser und rührt 1 Stunde. Zur Aufarbeitung verdünnt man mit eiskaltem Ether, schüttelt nacheinander mit eiskalter 10%iger Schwefelsäure, Natriumhydrogencarbonatlösung und Sole, trocknet über MgSO₄ und dampft im Vakuum ein. Das so erhaltene ölige 9-Tosylat wird in 120 ml Dimethysulfoxid gelöst, mit 9 g Kaliumnitrit versetzt und 3 Stunden auf 80° C erhitzt. Man verdünnt dann mit Wasser, extrahiert mit Ether, wäscht den Extrakt mit Sole, trocknet über MgSO₄ und dampft im Vakuum ein. Den Rückstand reinigt man durch Chromatographie an Kieselgel mit Hexan / 0-50 % Ethylacetat als Elutionsmittel und erhält 1,63 g der Titelverbindung als Öl.
IR (CHCl₃): 3605, 3460, 2935, 1745, 1022, 975 / cm.

### Beispiel 4

### (5Z, 13E)-(9R, 11R, 15S)-15-Gyclohexyl-9-fluor-11,15-dihydroxy-3-oxa-16, 17, 18, 19, 20-pentanor-5,13-prostadiensäure

Zu einer Lösung von 1,31 g (5Z, 13E)-(9S, 11R, 15S)-15-Cyclohexyl-9-hydroxy-3-oxa-11,15-bis-(tetrahydropyran-2-yloxy)-16, 17, 18, 19, 20-pentanor-5,13-prostadiensäure-tert.-butylester in 20 ml Methylenchlorid und 0,5 ml Pyridin gibt man bei -70° C 0,3 ml Diethylaminoschwefeltrifluorid (DAST) und nach 15 Minuten nochmals 0,1 ml DAST. Nach weiteren 15 Minuten wird mit 50 ml 5%iger Natriumhydrogencarbonatlösung versetzt, das Kältebad enfernt, 10 Minuten bei 20' C kräftig gerührt, dann mit Methylenchlorid extrahiert, der Extrakt mit Sole gewaschen, über MgSO₄ getrocknet und im Vakuum eingedampft. Den Rückstand rührt man 24 Stunden bei 20° C mit 20 ml einer Mischung aus Essigsäure/Wasser/Tetrahydrofuran (65/35/10), dampft nach Zugabe von Toluol im Vakuum ein und reinigt das Rohprodukt durch Chromatographie an Kieselgel mit Toluol/0-10 % Isopropanol. Man erhält (5Z, 13E)-(9R, 11R, 15S)-15-Cyclohexyl-9-fluor-11,15-dihydroxy-3-oxa-16, 17, 18, 19, 20-pentanor-5,13-prostadiensäure-tert.-butylester, welcher in Analogie zu Beispiel 9 verseift wird. Man erhält 52 mg der Titelverbindung als Öl.
IR (CHCl₃): 3600, 3410, 2930, 1740, 1022, 977 / cm.

## Patentansprüche

1. 9-Halogen-(Z)-Prostaglandinderivate der Formel I 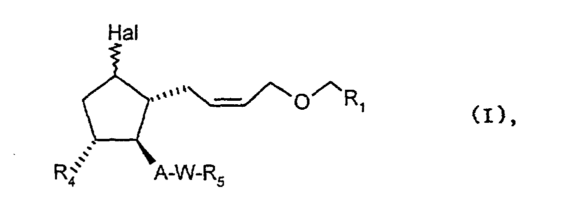 worin
Hal ein α- oder β-ständiges Chlor- oder Fluoratom ist,
R₁ den Rest CH₂OH oder 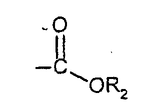 bedeutet, mit R₂ in der Bedeutung eines Wasserstoffatoms, eines Alkyl-, Cycloalkyl-, Aryl- oder heterocyclischen Restes oder R₁ den Rest 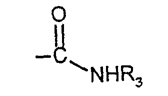 bedeutet, mit R₃ in der Bedeutung eines Säurerestes oder des Restes R₂ und
A eine -CH₂-CH₂-, eine trans-CH=CH- oder eine -C≡C-Gruppe ist,
W eine freie oder funktionell abgewandelte Hydroxymethylengruppe oder eine freie oder funktionell abgewandelte 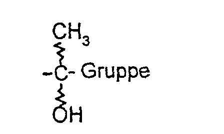 bedeutet, wobei die jeweiligen OH-Gruppen α- oder β-ständig sein können,
R₄ eine freie oder funktionell abgewandelte Hydroxygruppe ist,
R₅ eine Cycloalkylgruppe ist, die 3-10 Kohlenstoffatome im Ring enthält, und falls R₂ die Bedeutung eines Wasserstoffatoms hat, deren Salze mit physiologisch verträglichen Basen bedeutet und deren Cyclodextrinchlathrate.

2. Verfahren zur Herstellung der 9-Halogen-(Z)-prostaglandinderivate der Formel I gemäß Anspruch 1, **dadurch gekennzeichnet, daß** man in an sich bekannter Weise eine Verbindung der Formel II, 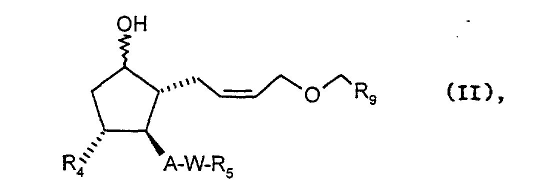 worin die 9-OH-Gruppe α-oder β-ständig sein kann und
R9 den Rest 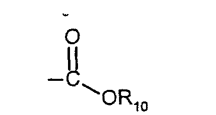 mit R₁₀ in der Bedeutung von Alkyl, Cycloalkyl, Aryl oder heterocyclischer Rest oder den Rest 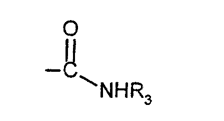 mit R₃ in der Bedeutung eines Säurerestes, eines Alkyl-, Cycloalkyl-, Aryl- oder eines heterocyclischen Restes darstellt und A und R₅ die bereits oben angegebenen Bedeutungen haben, nach vorherigem Schutz freier OH-Gruppen in R₄ und W
a) über einen intermediären Sulfonsäureester mit einem Halogenid der allgemeinen Formel III,
R₈―X (III),
worin R₈ die Bedeutung Lithium, Natrium, Kalium oder Tetraalkyl- bzw. Trialkylbenzyl-ammonium mit Alkyl als gesättigten C₁-C₆-Rest und X die Bedeutung Fluor oder Chlor hat oder
b) mit dem Reagenz Diethylaminoschwefeltrifluorid (DAST) zu Verbindungen der Formel I, worin Hal ein α- oder β-ständiges Fluoratom ist oder mit Tetrachlorkohlenstoff bzw. Hexachlorethan/Triphenylphosphin zu Verbindungen der Formel I, worin Hal ein α- oder β-ständiges Chloratom ist, umsetzt und anschließend in beliebiger Reihenfolge geschützte Hydroxygruppen freisetzt und/oder freie Hydroxygruppen verestert oder verethert und/oder Doppelbindungen hydriert und/oder eine veresterte Carboxylgruppe 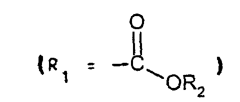 verseift und/oder eine freie Carboxylgruppe (R₂ = H) in ein Amid 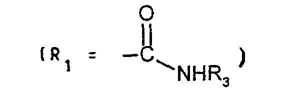 oder Salz überführt und/oder eine freie oder veresterte Carboxylgruppe 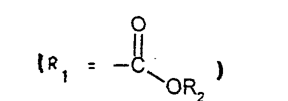 reduziert und/oder eine freie Carboxylgruppe (R₂ = H) in ein Salz überführt.

3. Arzneimittet, bestehend aus einer oder mehreren Verbindungen gemäß Anspruch 1 und üblichen Hilfsund Trägerstoffen.

## Claims

1. 9-Halo-(Z)-prostaglandin derivatives of the formula I 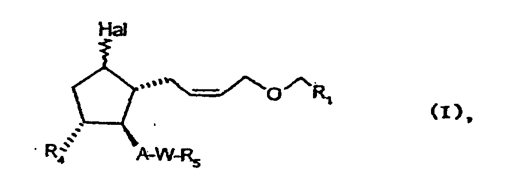 in which
Hal is an α- or β-chlorine or fluorine atom,
R₁ is the radical CH₂OH or 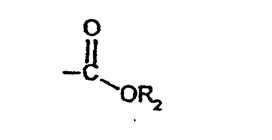 with R₂ meaning a hydrogen atom, an alkyl, cycloalkyl, aryl or heterocyclic radical or R₁ is the radical 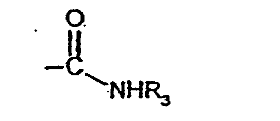 with R₃ meaning an acid residue or the radical R₂ and
A is a -CH₂-CH₂-, a trans-CH=CH- or a -C≡C- group,
W is a free or functionally modified hydroxymethylene group or a free or functionally modified 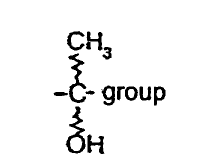 where the respective OH groups may be α or β,
R₄ is a free or functionally modified hydroxyl group,
R₅ is a cycloalkyl group which contains 3-10 carbon atoms in the ring and, if R₂ has the meaning of a hydrogen atom, the salts thereof with physiologically tolerated bases, and the cyclodextrin clathrates thereof.

2. Process for preparing the 9-halo-(Z)-prostaglandin derivatives of the formula I according to Claim 1, **characterized in that**, in a manner known per se, a compound of the formula II 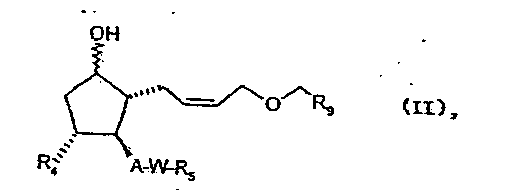 in which the 9-OH group may be α or β, and
R₉ is the radical 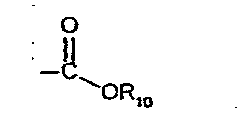 with R₁₀ meaning alkyl, cycloalkyl, aryl or heterocyclic radical or the radical 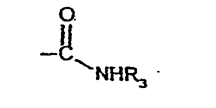 with R₃ meaning an acid residue, an alkyl, cycloalkyl, aryl or heterocyclic radical, and A and R₅ have the meanings already indicated with alkyl as saturated C₁-C₆ radical and X having the meaning of fluorine or chlorine, or
b) with the reagent diethylaminosulphur trifluoride (DAST) to give compounds of the formula I in which Hal is an α- or β-fluorine atom, or with tetrachloromethane or hexachloroethane/triphenylphosphine to give compounds of the formula I in which Hal is an α- or β-chlorine atom, and subsequently, in any sequence, liberating protected hydroxyl groups and/or esterifying or etherifying free hydroxyl groups and/or hydrogenating double bonds and/or hydrolysing an esterified carboxyl group 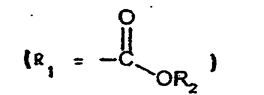 and/or converting a free carboxyl group (R₂ = H) into an amide 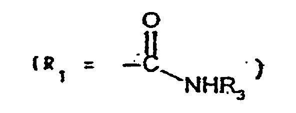 or salt, and/or reducing a free or esterified carboxyl group 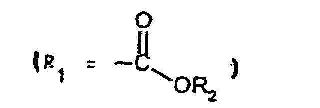
and/or converting a free carboxy group into a salt.

3. Medicament consisting of one or more compounds according to Claim 1 and conventional excipients and carriers.

## Revendications

1. Dérivés de 9-halogéno-(Z)-prostaglandines de formule I 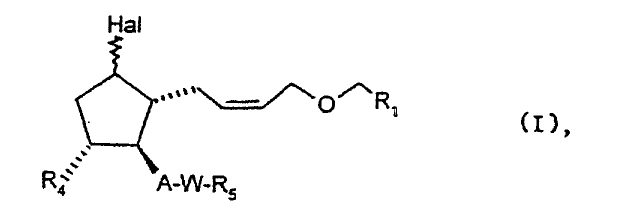 dans laquelle
Hal est un atome de chlore ou de fluor en position α ou β,
R₁ représente le radical CH₂OH ou 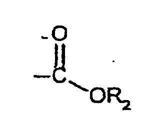 R₂ représentant un atome d'hydrogène, un radical alkyle, cycloalkyle, aryle ou hétérocyclique, ou R₁ représente le radical 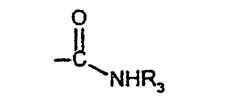
R₃ représentant un reste d'un acide ou le radical R₂ et
A étant un groupe -CH₂-CH₂-, un groupe trans-CH=CH-ou -C≡C-,
W représente un groupe hydroxyméthylène libre ou fonctionnellement modifié, ou un groupe 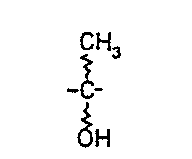 fonctionnellement modifié, les groupes OH pouvant être chacun en position α ou β,
R₄ est un groupe hydroxy libre ou fonctionnellement modifié,
R₅ est un groupe cycloalkyle qui contient de 3 à 10 atomes de carbone, et si R₂ représente un atome d'hydrogène, leurs sels avec des bases physiologiquement acceptables de tels composés et leurs clathrates avec des cyclodextrines.

2. Procédé pour la préparation des dérivés de 9-halogéno-(Z)-prostaglandines de formule I selon la revendication 1, **caractérisé en ce qu'**on fait réagir d'une façon connue en soi un composé de formule II, 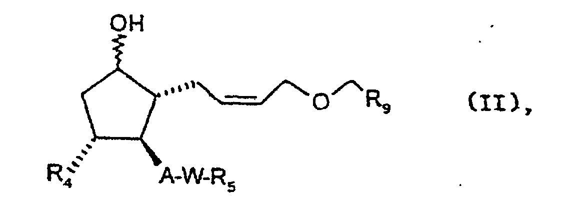 dans laquelle le groupe 9-OH peut être en position α ou β et
R₉ représente le radical 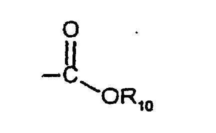 R₁₀ représentant un radical alkyle, cycloalkyle, aryle ou hétérocyclique, ou le radical 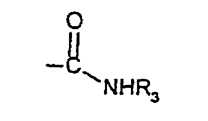 R₃ représentant un reste d'un acide ou un radical alkyle, cycloalkyle, aryle ou hétérocyclique, et
A et R₅ ont les significations déjà données plus haut,
après protection préalable des groupes OH libres dans R₄ et W
a) en passant par un ester d'acide sulfonique intermédiaire, avec un halogénure de formule générale III,
R₈-X (III)
dans laquelle R₈ représente le lithium, le sodium, le potassium ou un ion tétraalkyl- ou trialkylbenzylammonium, le fragment alkyle étant un radical en C₁-C₆ saturé et X représentant le fluor ou le chlore, ou
b) avec le réactif trifluorure de diéthylaminosoufre (DAST), pour aboutir à des composés de formule I dans lesquels Hal est un atome de fluor en position α ou β, ou avec du tétrachlorure de carbone ou de l'hexachloréthane/ triphénylphosphine pour aboutir à des composés de formule I dans lesquels Hal représente un atome de chlore en position α ou β, et ensuite, dans un ordre quelconque, on libère des groupes hydroxy protégés et/ou on estérifie ou éthérifie des groupes hydroxy libres et/ou on soumet des doubles liaisons à une hydrogénation et/ou on saponifie un groupe carboxy estérifié 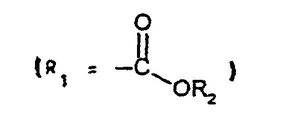 et/ou on convertit un groupe carboxy libre (R₂ = H) en un amide 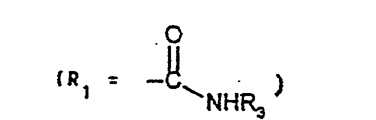
ou un sel et/ou on réduit un groupe carboxy libre ou estérifié 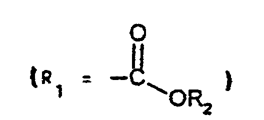 et/ou on convertit en un sel un groupe carboxy libre (R₂ = H).

3. Médicament constitué d'un ou plusieurs composés selon la revendication 1 et d'adjuvants et véhicules usuels.
